# EUROPÄISCHE PATENTSCHRIFT

(11) **EP 2 827 786 B1**
(45) Veröffentlichungstag und Bekanntmachung des Hinweises auf die Patenterteilung: **18.05.2016**
(21) Anmeldenummer: 13713746.9
(22) Anmeldetag: 20.03.2013
(51) Int. Cl.: A61B 17/32, A61B 17/3207, F16C 23/00

(54) **CHIRURGISCHES FRÄSINSTRUMENT**
SURGICAL MILLING INSTRUMENT
INSTRUMENT CHIRURGICAL DE FRAISAGE

(30) Priorität: 21.03.2012 DE 102012005536
(43) Veröffentlichungstag der Anmeldung: 28.01.2015
(73) Patentinhaber: OLYMPUS WINTER & IBE GMBH, 22045 Hamburg (DE)
(72) Erfinder: SCHÖLER, Uwe, 22955 Hoisdorf (DE); ZWEIBRÜCK, Dido Arnim, 22955 Hoisdorf (DE); SCHEEL, Andre, 21395 Tespe (DE)
(74) Vertreter: Hausfeld, Norbert
(86) Internationale Anmeldenummer: PCT/EP2013/000839
(87) Internationale Veröffentlichungsnummer: WO 2013/139473

(56) Entgegenhaltungen:
- EP-A1- 0 541 377
- EP-A1- 0 807 413
- US-A- 4 603 694
- US-A1- 2009 048 485

## Beschreibung

Die Erfindung betrifft ein chirurgisches Fräsinstrument der im Oberbegriff des Anspruches 1 genannten Art sowie ein Rohrmesser des Fräsinstrumentes und ein Verfahren zur Herstellung des Rohrmessers.

Ein gattungsgemäßes Fräsinstrument ist aus der EP 0 807 413 A1 bekannt auf welcher der Oberbegriff von Anspruch 1 beruht. Es dient zum fräsenden Abtragen von Körpergewebe aller Art und wird insbesondere endoskopisch eingesetzt, wozu es mit einem langgestreckten Schaftrohr geeignet ist.

Die Rohrmesser sind an einem Hauptkörper befestigt, und zwar das äußere Rohrmesser drehfest und das innere Rohrmesser drehangetrieben. Dazu ist im Hauptkörper z. B. ein Elektromotor vorgesehen. Die Länge der Schaftrohre ist den chirurgischen Verhältnissen angepasst, z. B. dem Arbeiten in einem Gelenk oder im Bauchraum. Das innere Rohrmesser dreht sich mit hoher Drehzahl. Dabei werden die Schneidfenster in den Schaftrohren relativ zueinander bewegt. In die Schneidfenster eintretendes Gewebe wird durch Scherbewegung der Ränder der Schneidfenster zueinander abgeschnitten.

Dazu müssen die Rohrmesser möglichst eng aufeinander gleiten und bedürfen zum langfristigen sicheren Betrieb bei hoher Drehzahl einer guten Lagerung aneinander. Dabei sind Lager zwischen den zylindrischen Schaftrohren vorgesehen. Im Bereich der halbkugelförmigen Endstücke ist eine axiale Lagerung der Schaftrohre aneinander erforderlich.

Nach dem Stand der Technik liegen zur axialen Lagerung die halbkugelförmigen Endstücke vollflächig aufeinander. Das ergibt zwar eine gute axiale Lagerung aber auch eine radiale Lagerung im Kugelbereich.

Eine zusätzliche radiale Lagerung im distalen Endstück der Rohrmesser ist jedoch ungünstig, da sie zusammen mit den Lagerungen im Bereich des zylindrischen Schaftrohres eine Überbestimmung ergibt, welche zu Verschleiß und zu Klemmgefahr führt.

Ein weiterer Nachteil der bekannten Konstruktion ist die sehr kostenaufwändige Herstellung der hohlhalbkugelförmig ausgebildeten distalen Endstücke, die in hochgenauer Passform ausgebildet sein müssen.

Beim bekannten Herstellungsverfahren wird das Schaftrohr am distalen Ende durch Drücken in die Kugelform gezogen und dadurch bis auf ein kleines Loch, das sehr aufwändig materialauftragend verschlossen werden muss, geschlossen.

Die Aufgabe der vorliegenden Erfindung besteht darin, bei einem gattungsgemäßen Fräsinstrument die Probleme der axialen Lagerung kostengünstig zu verbessern.

Diese Aufgabe wird mit den Merkmalen des Kennzeichnungsteiles des Anspruches 1 sowie mit den Ansprüchen 7 und 8 gelöst.

Erfindungsgemäß ist in einem Endstück, die Achse des Schaftrohres umgebend, eine Einsatzplatte eingesetzt. Damit erübrigt sich das Schließen des beim Zudrücken des Rohres verbleibenden Loches. Da die Platte eingesetzt wird, lässt sie sich vom Rohr getrennt bearbeiten und kann sehr einfach in geeigneter Weise für die axiale Lagerung ausgebildet werden. Diese Arbeiten an der Platte lassen sich wesentlich einfacher vornehmen, wenn diese noch vom Rohrmesser getrennt ist.

Die Endstücke können mit ihrer Einsatzplatte genau halbkugelförmig ausgebildet sein, wobei sich dann im Bereich der Endstücke zusätzlich zur axialen Lagerung auch eine radiale Lagerung ergäbe, die jedoch unerwünscht ist. Daher soll dafür Sorge getragen werden, dass im Bereich der halbkugelförmigen distalen Endstücke nur axiale Anlage, aber keine radiale Anlage gegeben ist. Dazu sind vorteilhaft die Merkmale des Anspruches 2 vorgesehen. Wenn eine Einsatzplatte mit der Mitte ihres zum anderen Endstück liegenden Fläche über die Kugelfläche vorspringt, dann ergibt sich im Bereich dieser vorspringenden Mitte die axiale Anlage zwischen den beiden distalen Endstücken, wodurch sich eine sehr saubere axiale Lagerstelle ergibt und zwar ohne radiale Führung. Dabei kann diese Ausbildung der Einsatzplatte sowohl am äußeren Rohrmesser, als auch am inneren Rohrmesser vorgesehen sein. Am äußeren Rohrmesser muss die Platte dabei nach innen vorspringend und am inneren Rohrmesser nach außen vorspringend ausgebildet sein.

Vorteilhaft ist dabei gemäß Anspruch 3 die Innenfläche der Einsatzplatte eben ausgebildet. Wenn diese Einsatzplatte am äußeren Rohrmesser vorgesehen ist, so liefert die ebene Innenfläche eine gute Anlage für den vorspringenden Bereich der Einsatzplatte am inneren Rohrmesser, wobei jede radiale Führung in diesem Bereich vermieden wird.

Dabei ist vorteilhaft nach Anspruch 4 die Außenfläche der Einsatzplatte als Teil der Kugelfläche ausgebildet. Es ergibt sich also auf der Außenseite des Rohrmessers eine völlig ungestörte Kugelfläche, wodurch z. B. Verletzungen vermieden werden.

Vorteilhaft gemäß Anspruch 5 können bei dieser Ausbildung der Einsatzplatte mit ebener Innenfläche und kugelförmiger Außenfläche beide Einsatzplatten identisch ausgebildet sein, was die Lagerhaltung und die Kosten erleichtert.

Die Einsatzplatte kann weitgehend beliebige Umfangsform haben, z. B. viereckig. Das Loch im Endstück, das die Einsatzplatte aufnehmen soll, muss dann entsprechend hergestellt werden. Vorzugsweise gemäß Anspruch 6 ist dieses Loch als konzentrisch zur Achse des zugehörigen Schaftrohres angeordnete Bohrung ausgebildet, wodurch die Herstellung des Loches in drehender Bearbeitung sehr stark vereinfacht wird. Auch die dazu passende Einsatzplatte mit zylinderförmiger Randfläche lässt sich kostengünstig herstellen.

Anspruch 7 schützt das Rohrmesser des Fräsinstrumentes.

Das erfindungsgemäße Rohrmesser kann auf unterschiedliche Weise hergestellt werden, vorzugsweise gemäß Anspruch 8. Dabei wird zunächst, vorzugsweise in rotierender Arbeitsweise, ein Ende des Schaftrohres in die Halbkugelform gedrückt, bis dort eine kleine Öffnung verbleibt, die kleiner ist, als das Loch, in das die Einsatzplatte eingesetzt werden soll. Anschließend wird dann das Loch für die Einsatzplatte, z. B. mit einem Bohrer eingebracht, um sodann die Einsatzplatte in dieses Loch einzusetzen und schließlich am Rand mit dem Schaftrohr zu verbinden, beispielsweise durch Verlöten, Verschweißen oder dergleichen.

In der Zeichnung ist die Erfindung beispielsweise und schematisch dargestellt. Es zeigen:
- Fig. 1: eine Seitenansicht eines erfindungsgemäßen Fräsinstrumentes,
- Fig. 2: einen Achsschnitt durch den vergrößerten distalen Endbereich des Fräsinstrumentes der Fig. 1 mit Darstellung des Bereiches der axialen Abstützung mittels zweier Einsatzplatten,
- Fig. 3 u. 4: eine Darstellung der Einsatzplatten in anderen Ausführungsformen,
- Fig. 5: eine Darstellung des Bereiches der axialen Anlage mit nur einer Einsatzplatte, und
- Fig. 6 - 8: in einem Achsschnitt durch den distalen Endbereich eines Rohrmessers drei Herstellungsschritte zur Ausbildung des distalen Endstückes.

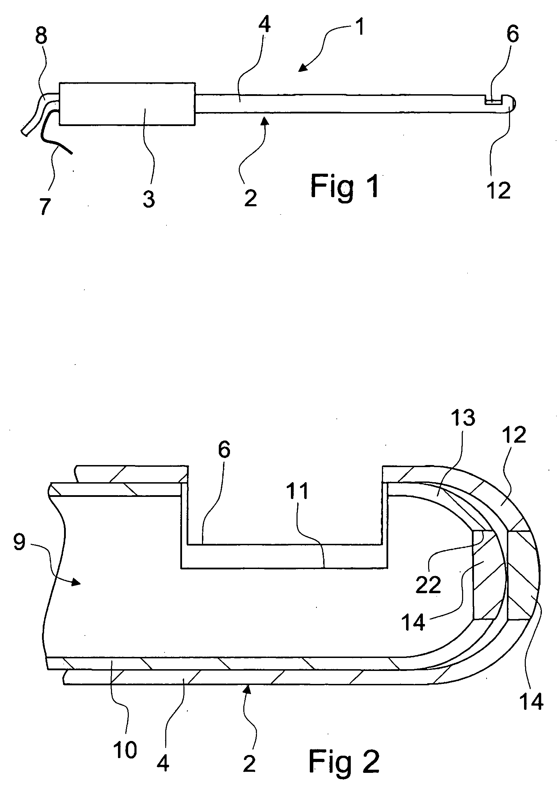

Fig. 1 zeigt ein erfindungsgemäßes Fräsinstrument 1 mit einem äußeren Rohrmesser 2, das an einem Hauptkörper 3 befestigt ist. Der Hauptkörper 3 kann als Handgriff verwendet werden und dazu entsprechend ausgebildet sein. Das äußere Rohrmesser 2 weist ein langgestrecktes zylindrisches Schaftrohr 4 und ein halbkugelförmig ausgebildetes distales Endstück 12 auf. Nahe des distalen Endstückes 12 ist im Rohrmesser 2 ein Schneidfenster 6 ausgebildet.

Im Hauptkörper 3 ist ein in der Fig. 1 nicht sichtbarer Elektromotor angeordnet, der über ein vom Hauptkörper 3 nach außen abgehendes Kabel 7 versorgt ist. Außerdem geht vom Hauptkörper 3 ein Schlauch 8 ab, der z. B. an eine geeignete Saugeinrichtung angeschlossen ist, um aus dem Inneren des Schaftrohres 4 abzusaugen.

Fig. 2 zeigt in vergrößertem Schnitt den distalen Endbereich des äußeren Rohrmessers 2. Es ist zu sehen, dass in diesem ein inneres Rohrmesser 9 angeordnet ist, das mit seinem zylindrischen Schaftrohr 10 im zylindrischen Schaftrohr 4 des äußeren Rohrmessers 2 in guter axialer Lagerung angeordnet ist. An der Stelle des Schneidfensters 6 im äußeren Rohrmesser 2 hat auch das innere Rohrmesser 9 ein dazu passendes Schneidfenster 11.

Das innere Rohrmesser 9 ist vom Motor im Hauptkörper 3 angetrieben und dreht sich um die gemeinsame Achse der beiden Rohrmesser 2 und 9. Dabei bewegen sich die Schneidfenster 6 und 11 relativ zueinander, so dass ihre Kanten eindringendes Gewebe abschneiden. Bei hoher Drehzahl ergibt sich eine sehr gute Fräswirkung. Abgefrästes Material gelangt durch die Schneidfenster 6, 11 ins Innere des inneren Rohrmessers 9 und wird von dort über den Saugschlauch 8 abgesaugt.

Fig. 2 zeigt, dass das distale Endstück 12 des äußeren Rohrmessers 2 ebenso wie das distale Endstück 13 des inneren Rohrmessers 9 hohlhalbkugelförmig ausgebildet sind. Wären die distalen Endstücke 12 und 13 so angeordnet, wie dies in der EP 0 807 413 A1 beschrieben ist, so würde die Innenfläche des äußeren Endstückes 12 genau der Außenfläche des inneren Endstückes 13 entsprechen und dieser flächig anliegen.

Mit einer solchen Kugelflächenanlage zwischen den beiden Endstücken 12 und 13 ergäbe sich eine axiale Abstützung, was notwendig und erwünscht ist, aber auch eine radiale Abstützung zwischen den Rohrmessern 2 und 9, was hier an den distalen Endstücken unerwünscht ist, da sich zusammen mit den sonstigen Lagerungen der Rohrmesser im Bereich der zylindrischen Schaftrohre 4 und 10 eine Überbestimmung ergäbe.

Die erfindungsgemäße Ausbildung gemäß Fig. 2 sorgt dafür, dass sich eine Abstützung der Rohrmesser 2 und 9 aneinander nur genau an einem Punkt ergibt, der in der Achse der Rohrmesser liegt. Die sonstigen Bereiche der Halbkugelflächen werden im Abstand gehalten, so dass sich in den Endstücken 12 und 13 keine axial stützende Anlage der Kugelflächen ergibt.

Im Ausführungsbeispiel der Fig. 2 wird das dadurch erreicht, dass in einem mittleren Bereich die Innenseite des äußeren Endstückes 12 eben ausgebildet ist. Dieser Bereich springt somit über die innere Kugelfläche des Endstückes 12 axial vor und sorgt für eine Abstandshaltung zwischen den Endstücken 12 und 13, auch wenn, wie dargestellt, die Außenfläche des inneren Endstückes 13 exakt kugelförmig bleibt.

Wie Fig. 2 zeigt, können dabei in diesem mittleren Bereich beide Endstücke 12 und 13 identisch ausgebildet sein.

Wie Fig. 2 ferner zeigt, sind die mittleren Bereiche der Endstücke 12 und 13 als identische Einsatzplatten 14 ausgebildet. Dadurch wird die Herstellung der mittleren Bereiche der Endstücke 12 und 13, die eine präzise Passform erfordern, stark vereinfacht. Die Einsatzplatten 14 können z.B. auf ganz andere Weise hergestellt werden, als die restlichen Teile der Rohrmesser.

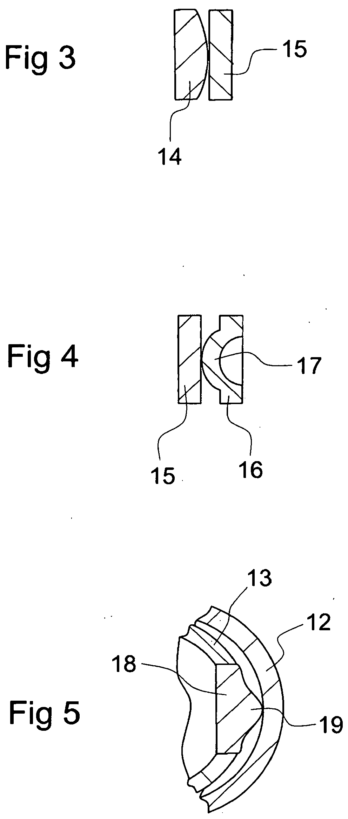

Fig. 3 zeigt bei ansonsten mit der Darstellung der Fig. 2 übereinstimmender, zur zeichnerischen Vereinfachung weggelassener Ausbildung nur die beiden Einsatzplatten, hier jedoch in anderer Ausbildung. Die Einsatzplatte 14 des inneren Rohrmessers 9 entspricht der Ausbildung der Fig. 2. Die Einsatzplatte 15 im äußeren Rohrmesser 2 ist an ihrer Innenfläche eben, wie auch die Einsatzplatte 14, jedoch in dieser Ausführungsform auch auf der Außenfläche eben, was hier als Beispiel einer möglichen Ausführungsform dargestellt wird.

Fig. 4 zeigt eine weitere Variante der Einsatzplatten, wobei die Einsatzplatte am inneren Rohrmesser die auf beiden Flächen ebene Platte 15 ist und die äußere Einsatzplatte 16 von völlig anderer Form ist. Sie springt nach innen mit einer Auswölbung 17 vor.

In Fig. 5 ist noch eine weitere Variante der axialen Lagerung dargestellt. Hier ist das äußere Endstück 12 ohne Einsatzplatte ausgebildet. Im inneren Endstück 13 ist eine Einsatzplatte 18 angeordnet, die einen deutlichen Vorsprung 19 in distaler Richtung aufweist. Bei dieser Ausführungsform kann auch das äussere Endstück 12 aus Gründen der Vereinfachung der Herstellung mit einer Einsatzplatte ausgebildet sein, die auf beiden Flächen Kugelform hätte.

Alle Ausführungsformen der axialen Abstützung, die in den Figuren 2 bis 5 dargestellt sind, ergeben denselben Effekt, nämlich eine punktförmige Anlage der Endstücke 12 und 13 genau auf der Rotationsachse des inneren Rohrmessers 9, wobei die restlichen Teile der Halbkugel ohne Anlage zwischen den Endstücken 12 und 13 bleibt.

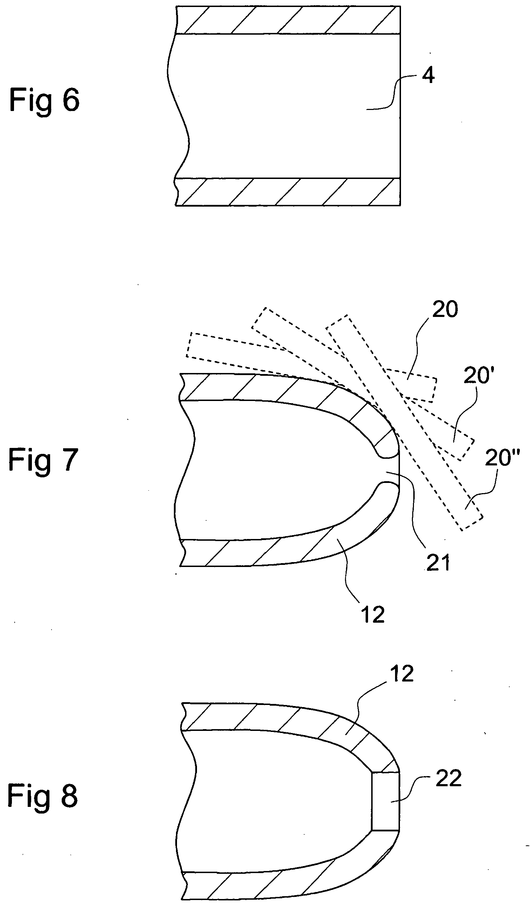

Die Figuren 6 und 8 dienen der Erläuterung eines vorteilhaften Verfahrens zur Herstellung des Rohrmessers 2, das auch zur Herstellung des Rohrmessers 9 verwendbar ist, das bis auf den Durchmesser identisch ausgebildet sein kann, wie dies die Fig. 2 zeigt.

Ausgegangen wird dabei von einem geraden Rohrstück, dessen distaler Endbereich in Fig. 6 gezeigt wird. Es handelt sich im dargestellten Beispiel um den Endbereich des zylindrischen Schaftrohres 4. Das Schaftrohr 4 ist z. B. mit der dargestellten geraden Schnittfläche von einem Endlosrohr abgeschnitten.

Das Rohrstück 4 wird z. B. auf einer Drehbank rotierend angetrieben und in seinem dargestellten Endbereich mit einem Drückwerkzeug rotierend verformt, so dass das dargestellte Ende im Durchmesser immer enger eingezogen wird, bis sich die in Fig. 7 dargestellte verrundete Form des Endstückes 12 ergibt. Zum Drücken kann z.B. ein in Fig. 7 dargestelltes gerades Werkzeug 20 verwendet werden, das allmählich über die Stellungen 20, 20', 20" immer mehr angewinkelt wird, um dabei das Rohr 4 aus der geraden Form gemäß Fig. 6 in die verrundete Form gemäß Fig. 7 zu drücken.

Bei dieser Herstellung verbleibt eine Öffnung 21, die durch Verschweißung oder ähnliche materialauftragende Technik verschlossen werden kann. Vorzugsweise wird jedoch wie in Fig. 8 dargestellt, in der Achse des Rohres 4 eine Bohrung 22 eingebracht, die in ihrem Durchmesser zum Außendurchmesser der Einsatzplatten, z. B. der Einsatzplatte 14 gemäß Fig. 2 passt. Darin kann dann eine Einsatzplatte 14 eingesetzt werden, wie dies in Fig. 2 dargestellt ist, und am Rand befestigt werden, z. B. durch Verschweißung.

Die Einsatzplatten 14, 15, 16 oder 18 sind an dem Punkt ihrer Berührung einem erhöhten Verschleiss ausgesetzt und bestehen daher vorteilhaft aus einem besoners verschleissfesten Material wie z.B Hartmetall oder Keramik.

## Patentansprüche

1. Chirurgisches Fräsinstrument mit zwei ineinander angeordneten Rohrmessern (2, 9), die jeweils ein zylindrisches Schaftrohr (4, 10), ein damit einstückig hohlhalbkugelförmig ausgebildetes distales Endstück (12, 13) und ein Schneidfenster (6, 11) aufweisen, wobei das äußere Rohrmesser (2) drehfest und das innere Rohrmesser (9) drehangetrieben an einem Hauptkörper (3) befestigt sind, **dadurch gekennzeichnet, dass** wenigstens eines der Endstücke (12, 13) in einem die Achse des zugehörigen Schaftrohres (4, 10) umgebenden Loch (22) eine an ihrem Rand mit dem Rand des Loches (22) verbundene Einsatzplatte (14, 15, 16, 18) aufweist.

2. Fräsinstrument nach Anspruch 1, **dadurch gekennzeichnet, dass** die Einsatzplatte (14, 15, 16, 18) mit der Mitte ihrer zum anderen Endstück liegenden Fläche über die Kugelfläche (12, 13) des einen Endstücks vorspringend ausgebildet ist.

3. Fräsinstrument nach Anspruch 2, **dadurch gekennzeichnet, dass** die Innenfläche der Einsatzplatte (14, 15, 18) eben ausgebildet ist.

4. Fräsinstrument nach Anspruch 3, **dadurch gekennzeichnet, dass** die Außenfläche der Einsatzplatte (14) als Teil der Kugelfläche ausgebildet ist.

5. Fräsinstrument nach Anspruch 4, **dadurch gekennzeichnet, dass** die Einsatzplatten (14) beider Rohrmesser (2, 9) identisch ausgebildet sind.

6. Fräsinstrument nach einem der vorhergehenden Ansprüche, **dadurch gekennzeichnet, dass** das Loch als konzentrisch zur Achse des zugehörigen Schaftrohres (4, 10) angeordnete Bohrung (22) ausgebildet ist.

7. Rohrmesser eines Fräsinstrumentes nach einem der vorhergehenden Ansprüche, **dadurch gekennzeichnet, dass** das Rohrmesser ein zylindrisches Schaftrohr (4, 10) und ein damit einstückig hohlhalbkugelförmig ausgebildetes distales Endstück (12, 13) aufweist, und wobei das Endstück (12, 13) in einem die Achse des zugehörigen Schaftrohres (4, 10) umgebenden Loch (22) eine an ihrem Rand mit dem Rand des Loches (22) verbundene Einsatzplatte (14, 15, 16, 18) aufweist.

8. Verfahren zur Herstellung eines Rohrmessers nach Anspruch 7, **dadurch gekennzeichnet, dass** ein Ende des Schaftrohres (4) zur Halbkugelform gedrückt wird, bis eine Öffnung (21) verbleibt, die kleiner ist, als die Fläche des Loches (22), das dann das Loch (22) eingebracht und in dieses die Einsatzplatte (14) eingesetzt und schließlich diese am Rand befestigt wird

## Claims

1. A surgical milling instrument comprising two tubular cutters (2, 9) arranged inside one another, which in each case have a cylindrical shaft tube (4, 10), a distal end piece (12, 13) integrally formed therewith in a hollow hemispherical manner, and a cutting window (6, 11), wherein the outer tubular cutter (2) is non-rotatably mounted on a main body (3) and the inner tubular cutter (9) is mounted in a rotationally driven manner thereon,
**characterised in that** at least one of the end pieces (12, 13) in a hole (22) surrounding the axis of the associated shaft tube (4, 10) has an insert plate (14, 15, 16, 18), which is connected on the edge thereof to the edge of the hole (22).

2. The milling instrument according to claim 1, **characterised in that** the insert plate (14, 15, 16, 18) is embodied so that the centre of its surface, which is located towards the other end piece, protrudes beyond the spherical surface (12, 13) of the one end piece.

3. The milling instrument according to claim 2, **characterised in that** the inner surface of the insert plate (14, 15, 18) is embodied so as to be flat.

4. The surgical milling instrument according to claim 3, **characterised in that** the outer surface of the insert plate (14) is embodied as part of the spherical surface.

5. The milling instrument according to claim 4, **characterised in that** the insert plates (14) of both tubular cutters (2, 9) are embodied identically.

6. The milling instrument according to any one of the preceding claims, **characterised in that** the hole is embodied as bore (22), which is arranged concentrically to the axis of the associated shaft tube (4, 10).

7. A tubular cutter of a milling instrument according to any one of the preceding claims, **characterised in that** the tubular cutter has a cylindrical shaft tube (4, 10) and a distal end piece (12, 13) integrally formed therewith in a hollow hemispherical shape, and wherein the end piece (12, 13) in a hole (22) surrounding the axis of the associated shaft tube (4, 10) has an insert plate (14, 15, 16, 18), which is connected on the edge thereof to the edge of the hole (22).

8. A method for producing a tubular cutter according to claim 7, **characterised in that** one end of the shaft tube (4) is pushed into the hemispherical shape until an opening (21) remains, which is smaller than the surface of the hole (22), that the hole (22) is then introduced and the insert plate (14) is inserted into said hole and said insert plate is finally mounted to the edge.

## Revendications

1. Instrument de fraisage chirurgical avec deux lames tubulaires (2, 9) agencées l'une dans l'autre et présentant respectivement une gaine cylindrique (4, 10), un embout distal (12, 13) de forme hémisphérique creuse formé d'un seul tenant avec la gaine, et une fenêtre de coupe (6, 11), la lame tubulaire extérieure (2) étant fixée à un corps principal (3) sans pouvoir tourner par rapport à celui-ci et la lame tubulaire intérieure (9) y étant montée de façon à pouvoir être entraînée en rotation, **caractérisé en ce que** l'un au moins des embouts (12, 13) présente, dans un trou (22) entourant l'axe de la gaine (4, 10) correspondante, une plaque rapportée (14, 15, 16, 18) dont le bord est joint au bord du trou (22).

2. Instrument de fraisage selon la revendication 1, **caractérisée en ce que** la plaque rapportée (14, 15, 16, 18) est conformée de façon à ce que la partie centrale de sa surface tournée vers l'autre embout fasse saillie au-delà de la surface sphérique (12, 13) de l'embout dans lequel elle est insérée.

3. Instrument de fraisage selon la revendication 2, **caractérisée en ce que** la face intérieure de la plaque rapportée (14, 15, 18) est plane.

4. Instrument de fraisage selon la revendication 3, **caractérisée en ce que** la face extérieure de la plaque rapportée (14) est conformée de façon à former une partie de la surface sphérique.

5. Instrument de fraisage selon la revendication 4, **caractérisée en ce que** les plaques rapportées (14) des deux lames tubulaires (2, 9) ont toutes deux une conformation identique.

6. Instrument de fraisage selon l'une des revendications précédentes, **caractérisé en ce que** le trou est réalisé sous forme d'alésage (22) concentrique à l'axe de la gaine (4, 10) correspondante.

7. Lame tubulaire d'un instrument de fraisage selon l'une des revendications précédentes, **caractérisée en ce que** la lame tubulaire présente une gaine cylindrique (4, 10) et un embout distal (12, 13) de forme hémisphérique creuse formé d'un seul tenant avec cette gaine, l'embout (12, 13) présentant, dans un trou (22) entourant l'axe de la gaine (4, 10) correspondante, une plaque rapportée (14, 15, 16, 18) dont le bord est joint au bord du trou (22).

8. Procédé de fabrication d'une lame tubulaire selon la revendication 7, **caractérisé en ce qu**' une extrémité de la gaine (4) est pressée en forme d'hémisphère jusqu'à réserver un orifice (21) plus petit que la surface du trou (22), le trou (22) étant alors formé dans cet orifice et la plaque rapportée (14) insérée dans ce trou puis fixée à son bord.
